# EUROPEAN PATENT SPECIFICATION

(11) **EP 2 490 585 B1**
(45) Date of publication and mention of the grant of the patent: **27.05.2020**
(21) Application number: 10776457.3
(22) Date of filing: 19.10.2010
(51) Int. Cl.: A61B 3/113, A61F 9/00

(54) **LIQUID DISPENCING WITH BLINK DETECTION**
FLÜSSIGKEITSAUSGABE MIT LIDSCHLAGERKENNUNG
DISTRIBUTION DE LIQUIDE AVEC DÉTECTION DES CLIGNEMENTS

(30) Priority: 12.10.2010 US 902575; 21.10.2009 US 253613 P
(43) Date of publication of application: 29.08.2012
(73) Proprietor: Johnson & Johnson Vision Care, Inc., Jacksonville, FL 32256 (US)
(72) Inventor: VOSS, Leslie, A., Jacksonville FL 32258 (US); MORLEY, Catie, A., St. Johns FL 32259 (US); HALL, Gary, S., Jacksonville FL 32259 (US)
(74) Representative: Kirsch, Susan Edith
(86) International application number: PCT/US2010/053132
(87) International publication number: WO 2011/049911

(56) References cited:
- EP-A2- 1 493 410
- WO-A1-2004/028421
- WO-A2-99/52479

## Description

### FIELD OF USE

This invention describes a device for dispensing liquids or mists into the eye, and, more specifically, in some embodiments, a device that dispenses a spray or mist into the eye based upon detection of a blink.

### BACKGROUND

It has been known to dispense a liquid or a mist into an eye using many different devices. However, although many devices result with a liquid entering the eye, the experience of getting the liquid into the eye is generally less than satisfactory.

Devices for self dispensing liquids typically require that a user hold the eyelids open to fight the blink reflex. This contention inhibits easy application of the desired fluids. Some automated devices pull down on one lid, or encapsulate the eye area to stop the lids from closing. This touch is damaging to makeup, and can lead to contamination of the device and the liquid entering the eye.

The dose from the system should consistently, without great user effort, dispense into the user's eye, not upon the eyelid or other part of the users face, and optimally should not touch the face in a manner that damages makeup or contaminates the device.

Some dispensing devices simulate a 'gun' and shoot a fluid in at the eye at a rate calculated to beat the blink reflex, however the speed and impact of the fluid seem to induce discomfort in the patient.

Other devices force the lids open in different manners through touching the cheek below the lid, and the eyebrow range above the upper lid, then spray the fluid into the eye. This forcing open of the lids is uncomfortable, and the unit itself becomes large and unwieldy. Any makeup worn by the consumer is smudged during the process and sometimes contaminates the dispenser and/or the dispensed liquid.

Misting of fluid over the entire eye or even the facial area is also feasible, but wets not only the eye, but undesirable surfaces such as the eyelid, forehead, and nose. Application of liquid to the eyelid is declared to also wet the eye by flowing into the eye, but results from this method are mixed, and the wetting of the lid itself is typically undesirable.

WO2004028421 relates to a method and apparatus for detecting the state of an eye. i. e.. whether the eye is open or closed. In particular, the document relates to a method and apparatus for controlling timing of ocular intervention required in diagnosis, prevention or treatment of an ophthalmic condition, disorder or disease.

### SUMMARY

Accordingly, the present invention includes a device as defined in appended claim 1 for accurately and cleanly dispensing a liquid or mist into an eye. The present invention automatically senses a blink and dispenses the liquid in a timely fashion following the blink to allow the liquid to enter the eye before the eye may blink again. By using the window just after the blink, the present invention consistently provides enough time to dispense in to the eye and also dispense at a rate which is adequately slow application of fluid into the eye to maintain the inertial impact of the fluid on the eye at a comfortable level.

### DESCRIPTION OF THE DRAWINGS

FIG. 1 illustrates a dispensing device sensing a closed eye according to some embodiments of the present invention.
FIG. 2 illustrates a dispensing device sensing an open eye according to some embodiments of the present invention.
FIG. 3 illustrates an eye with an alignment apparatus.
FIG. 4 illustrates an exemplary apparatus for sensing a state of an open or closed eye.
Fig. 5 illustrates a controller that may be used to implement some embodiments of the present invention.

### DETAILED DESCRIPTION OF THE INVENTION

The present invention includes a device for dispensing a liquid or a mist into an eye. The device for dispensing a liquid or a mist into an eye includes a detection method to determine when a blink has been completed. Dispensing is timed to an

interval based upon a determination of when an eye into which the liquid will be dispensed opens and closes, such as, for example, in a consciously induced eye blink or a natural eye blink. The blink is utilized to determine whether an eye is known to be open whereby a liquid can be dispensed before the patient closes the eye.

In some embodiments the device includes features to minimize the need for facial contact during liquid application. Additional embodiments include alignment features to assure proper alignment of the device to the eye. For example, in some embodiments, the dispensing device includes protrusions that rest on the eyebrow, and have a small diameter hole for the user to look through. When the user is able to see through the hole, proper alignment has been achieved).

Once the device is properly aligned with an eye, opening and closing of the eye is automatically determined by a sensor. A dispensing apparatus in logical communication with the sensor is programmed to dispense a liquid or mist into the eye according to the timing of an open cycle of a blink. The alignment process coupled with the blink detection eliminates the need for holding the lids open, touching the face, or contamination associated with regular facial touch. In addition, by dispensing based upon an opening motion of an eyelid, a dispenser according to the present invention, consistently wets the eye without wetting the eyelid or surrounding face.

In the following sections detailed descriptions of embodiments of the invention will be given. The description of both preferred and alternative embodiments are exemplary embodiments only, and it is understood that to those skilled in the art that variations, modifications and alterations may be apparent. It is therefore to be understood that said exemplary embodiments do not limit the scope of the underlying invention, which is defined by the appended claims.

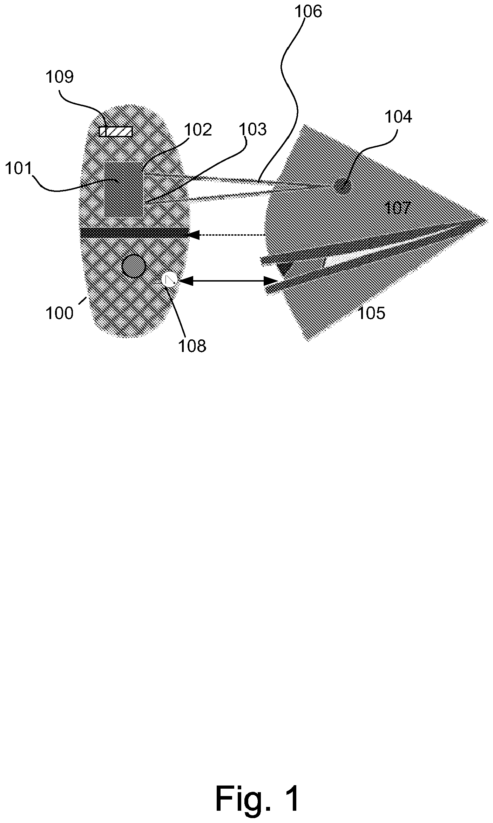

Referring now to Fig. 1, a liquid dispensing device 100 includes one or more electronic sensors 101 capable of sensing an open state or a closed state of an eye 105. The one or more sensors 101 include an emitter 102 and a detector 103. The emitter 102 emits a beam 106 which reflects off of a reflecting point 104 and back to the detector 103. As illustrated in Fig. 1, the reflecting point 104 is on the eyelid 107 of the eye. The beam 106 may include, for example one or more of: infra red light, visible light, ultrasonic wavelengths, or other wavelengths.

A processor, 109 receives input from one or both of the emitter 102 and the detector 103. Executable software may cause the processor to be
functional to calculate an amount of reflection of the beam 106. The software may be stored in a digital storage that is in logical communication with the processor. In some embodiments, the storage may be inherent with a microcontroller including the processor. A first range of an amount of reflection may correlate with a closed eye state, and a second amount of reflection may correlate with an open eye state.

In another aspect, a proximity sensor 108 may also include an emitter and a detector, and may be positioned such that a reflected beam may be used for the processor 109 to determine a relative distance between the liquid dispensing device 100 and a surface, such as the surface of an eye 105.

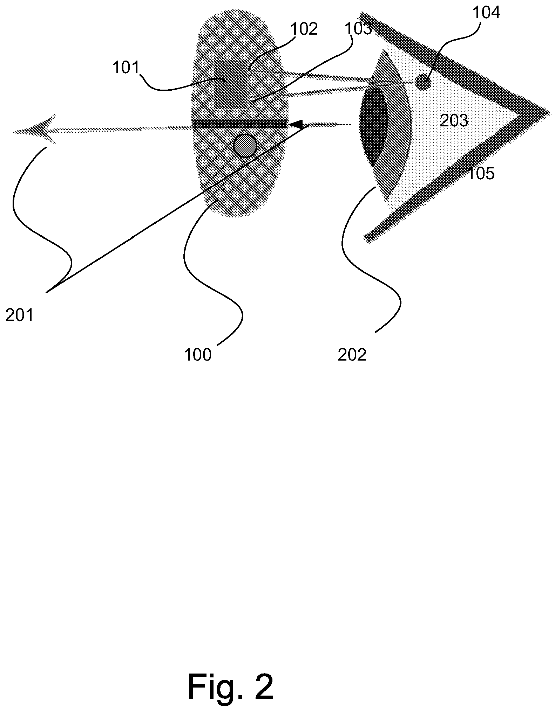

Referring now to Fig. 2, an open eye 105 provides for a reflecting point 104 on an open portion 203 of the eye 105, as opposed to the eyelid 107. The open portion of the eye 203 can include, for example, reflection of off the sclera or other portion of the eye. In some embodiments, a wavelength of an emitted beam is correlated with physical characteristics of the reflecting point 104. Reflection of the emitter beam 106 off of the eyelid will reflect back with a first set of reflection characteristics and reflection off of the open portion of the eye 203 with a second set of reflection characteristics. The reflection characteristics will be sensed by the detector 103.

In another aspect, of the present invention, alignment of the eye 105 with the liquid dispensing device 100 may be facilitated by a focal point of the emitter 102 being aligned with a predetermined portion of the eye, such as, for example: the sclera, iris, and pupil of the eye. Alignment is accomplished via a line of sight 201. When a pupil 202 is aligned with the line of sight 201, the sensor 101 is also properly aligned to sense an open state and a closed state of the eye 105. The device body includes the sensor 101 and dispenser such that when a patient aligns to a tubular cutout in the body which forms the line of sight 201 in the dispensing device, a position of the liquid dispensing device 100 creates a coaxial alignment between the line of sight and the center of the tube. The alignment establishes an angular and X-Y location of the pupil relative to the dispensing device 100.

In some embodiments, a line of sight can be combined with a positioning device which includes one or more alignment legs which press against the face and/or forehead.

In addition, in some embodiments, an audible signaling device may be included within a sensor or I electrical communication with the sensor. When the sensor measures a distance of the device from the eye the audible signaling device may signal (perhaps by click, tone, sound, or vibration,) that it is within an acceptable Z positional range from the eye for an optimal dose. It is expected that this range will be relatively wide (in the 2-5 mm range) so any of the sensors noted above as able to detect the blink could also be used to detect a distance from the eye to the dispensing device 100.

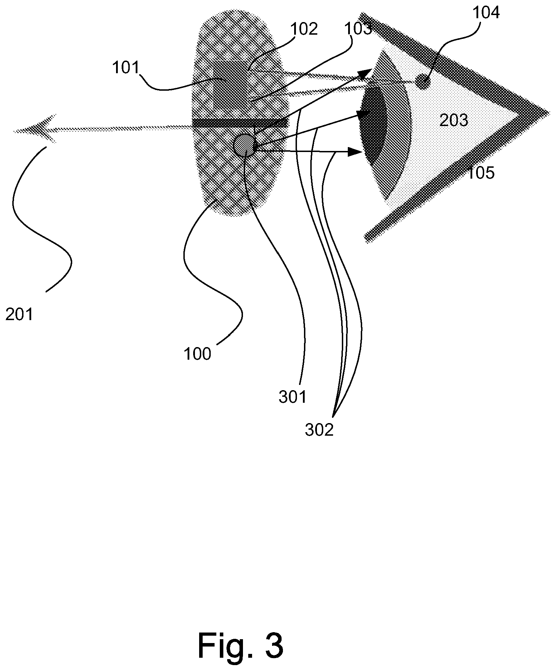

Referring now to Fig. 3, a dispensing nozzle 301 will dispense a liquid via sprays 302 or mist (not illustrated) when the sensor 101 senses that the eye 105 is in an open state. The open state is determined by the nature of the beam 106 sensed by the detector 103. Preferred embodiments, dispense a liquid spray 302 based upon timing that indicates that the eyelid 107 is involved in an opening cycle. Dispensing sprays 302 during an opening cycle of an eye can be accomplished such that the patient cannot physically blink to close the eye before the liquid is dispensed. The spray may be a liquid stream or a mist.

In another aspect, a liquid spray 302 may be dispensed based upon a range of distance of the proximity sensor from the eye 105.

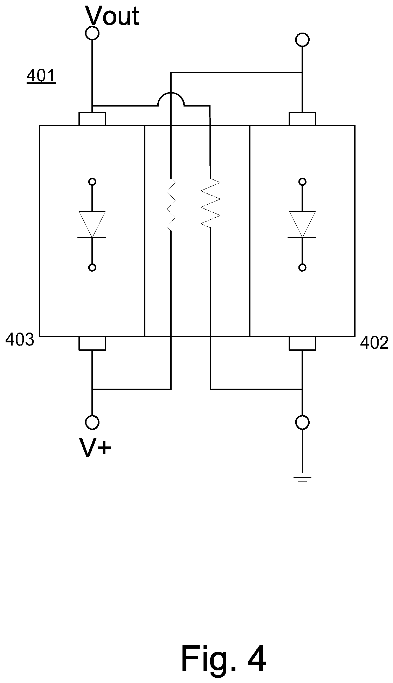

Referring now to Fig. 4, an example of a sensing device used to detect an open state and a closed state of an eyelid is illustrated. During laboratory tests, the exemplary sensor 301 was connected to an oscilloscope (not shown) and positioned proximate to the eye. In some experiments, the sensor was positioned approximately 6 millimeters from the eye. The oscilloscope recorded that that the sensor 301 successfully provided a logic signal indicating that the sensor 301 accurately detected an open state versus a closed state of an eye. The detector 401 included an emitter 402 and a detector 403. The sensor accurately detected transition from a first state of an eye, such as an open eyelid state to a second state of an eye, such as a closed eyelid state.

According to the present invention, the detector 401 is placed in logical communication with an automated dispensing unit capable of dispensing a predetermined amount of a liquid into the eye. Dispensing units are currently known and available which can receive an electrical signal based upon the logic of a sensing device 101 and activates the dispensing of a dose of liquid into an eye positioned proximate to the dispensing unit. For example, an automated dispenser may include an
electrically powered pump which dispenses a pulsatile liquid dose of a medicament into an eye. The electrically powered pump will respond quickly enough to allow a pulsatile dose to enter the eye before the eye can respond to the entry of the liquid into the eye.

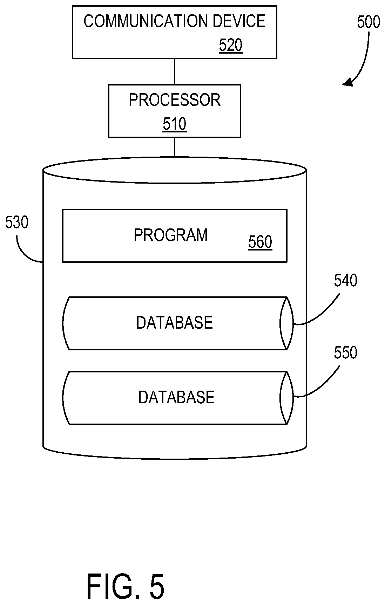

Referring now to Fig. 5 a controller 500 is illustrated that may be used in some embodiments of the present invention. The controller 600 includes a processor 610, which may include one or more processor components coupled to a communication device 620. In some embodiments, a controller 600 can be used to receive a logical indication that an eye is in a first state or a second state and transmit energy to liquid dispenser at a time appropriate to dispense a liquid or mist into the eye, based upon the transition from a first state to a second state.

The controller can include one or more processors, coupled to a communication device configured to communicate energy via a communication channel. The communication device may be used to electronically control, for example, one or more of: timing of liquid dispensing; an amount of liquid dispensed; duration of a dispensing motion, tracking a number of dispensing actions, tracking chronological dispensing patterns or other actions related to the dispensing.

The processor 410 is also in communication with a storage device 630. The storage device 430 may comprise any appropriate information storage device, including combinations of magnetic storage devices (e.g., magnetic tape and hard disk drives), optical storage devices, and/or semiconductor memory devices such as Random Access Memory (RAM) devices and Read Only Memory (ROM) devices.

The storage device 430 can store a program 440 for controlling the processor 410. The processor 410 performs instructions of the program 440, and thereby operates in accordance with the present invention. For example, the processor 410 may receive information descriptive of liquid to be dispensed, dispensing amounts, dispensing patterns, and the like.

The present invention, as described above and as further defined by the claims below, provides methods of providing a liquid dispenser with blink detecting mechanisms.

## Claims

1. An apparatus (100) for dispensing liquid into an eye, the apparatus (100) comprising:
an emitter (102) for emitting a light beam towards an eye (105);
a detector (103) for detecting light reflected off of an open eye (105) while the detector (103) is positioned proximate to the eye (105);
an automated dispenser for dispensing a liquid (302) towards the eye (105);
a processor (109) in logical communication with the detector (103), the processor (109) functional to coordinate dispensing of the liquid (302) towards the eye (105) based upon a detection of light reflected off of an eye (105);
wherein the apparatus comprises a line of sight comprising a tubular cutout in a body of the apparatus (100).

2. The apparatus (100) of claim 1 additionally including a digital storage storing executable software operative with the processor (109) to cause the processor (109) to be functional to coordinate dispensing of the liquid (302) towards the eye (102) based upon a detection of light reflected off of an eye (105).

3. The apparatus (100) of claim 2 additionally comprising a proximity sensor (108) in logical communication with the processor (109), said proximity sensor (108) communicating a logical signal based upon the sensors proximity to a surface.

4. The apparatus (100) of claim 3 additionally comprising a dispensing nozzle (301) in fluid communication with the automated dispenser and functional to dispense liquid (302) via a spray.

5. The apparatus (100) of claim 3 additionally comprising a dispensing nozzle (301) in fluid communication with the automated dispenser and functional to dispense liquid (302) via a mist.

6. The apparatus (100) of any one of claims 3 to 5 additionally comprising an audible signaling device in electrical communication with the proximity sensor (108), said audible signaling device capable of emitting an audible signal based upon a signal from the proximity sensor (108).

7. The apparatus (100) of any one of claims 3 to 6, wherein the processor (109) is functional to receive a signal from the detector (102) indicating an eye (105) is in an open state and generate a logical signal to the dispenser based upon the receipt of the signal indicating an eye (105) is in an open state.

8. The apparatus (100) of claim 7 wherein the dispenser dispenses a liquid (302) based upon the receipt of the signal indicating an eye (105) is in an open state.

## Patentansprüche

1. Vorrichtung (100) zum Abgeben von Flüssigkeit in ein Auge, wobei die Vorrichtung (100) umfasst:
einen Emitter (102) zum Emittieren eines Lichtstrahls in Richtung eines Auges (105);
einen Detektor (103) zum Detektieren von Licht, das von einem geöffneten Auge (105) reflektiert wird, während der Detektor (103) in der Nähe des Auges (105) positioniert wird;
eine automatisches Abgabegerät zum Abgeben einer Flüssigkeit (302) in Richtung des Auges (105);
einen Prozessor (109) in logischer Kommunikation mit dem Detektor (103), wobei der Prozessor (109) funktional ist, um Abgeben der Flüssigkeit (302) in Richtung des Auges (105) basierend auf einer Detektierung von Licht zu koordinieren, das von einem Auge (105) reflektiert wird;
wobei die Vorrichtung eine Sichtlinie, die einen rohrförmige Ausschnitt umfasst, in einem Körper der Vorrichtung (100) umfasst.

2. Vorrichtung (100) nach Anspruch 1, die zusätzlich einen digitalen Speicher einschließt, der mit dem Speicher (109) betriebsfähige ausführbare Software einschließt, um zu bewirken, dass der Prozessor (109) funktional ist, um Abgeben der Flüssigkeit (302) in Richtung des Auges (102) basierend auf einer Detektierung von Licht zu koordinieren, das von einem Auge (105) reflektiert wird.

3. Vorrichtung (100) nach Anspruch 2, die zusätzlich einen Näherungssensor (108) in logischer Kommunikation mit dem Prozessor (109) umfasst, wobei der Näherungssensor (108) ein logisches Signal basierend auf der Nähe des Sensors zu einer Oberfläche kommuniziert.

4. Vorrichtung (100) nach Anspruch 3, umfassend zusätzlich eine Abgabedüse (301) in Fließkommunikation mit dem automatisierten Abgabegerät, und funktional, um Flüssigkeit (302) als Spray abzugeben.

5. Vorrichtung (100) nach Anspruch 3, umfassend zusätzlich eine Abgabedüse (301) in Fließkommunikation mit dem automatisierten Abgabegerät, und funktional, um Flüssigkeit (302) als Nebel abzugeben.

6. Vorrichtung (100) nach einem der Ansprüche 3 bis 5, zusätzlich umfassend eine akustische Signalisiereinrichtung in elektrischer Kommunikation mit dem Näherungssensor (108), wobei die akustische Signalisiereinrichtung in der Lage ist, basierend auf einem Signal von dem Näherungssensor (108) ein akustisches Signal zu emittieren.

7. Vorrichtung (100) nach einem der Ansprüche 3 bis 6, wobei der Prozessor (109) funktional ist, um ein Signal von dem Detektor (102) zu empfangen, welches angibt, dass sich ein Auge (105) in einem geöffneten Zustand befindet, und ein logisches Signal an das Abgabegerät basierend auf dem Empfang des Signals zu generieren, welches angibt, dass sich ein Auge (105) in einem geöffneten Zustand befindet.

8. Vorrichtung (100) nach Anspruch 7, wobei das Abgabegerät basierend auf dem Empfang eines Signals, welches angibt, dass sich ein Auge (105) in einem geöffneten Zustand befindet, eine Flüssigkeit (302) abgibt.

## Revendications

1. Appareil (100) permettant de distribuer un liquide dans un œil, l'appareil (100) comprenant :
un émetteur (102) pour émettre un faisceau de lumière en direction d'un œil (105) ;
un détecteur (103) pour détecter une lumière réfléchie par un œil ouvert (105) pendant que le détecteur (103) est positionné à proximité de l'œil (105) ;
un distributeur automatisé pour distribuer un liquide (302) en direction de l'œil (105) ;
un processeur (109) en communication logique avec le détecteur (103), le processeur (109) étant fonctionnel pour coordonner la distribution du liquide (302) en direction de l'œil (105) sur la base d'une détection d'une lumière réfléchie par un œil (105) ;
l'appareil comprenant une ligne de visée comprenant une découpe tubulaire dans un corps de l'appareil (100).

2. Appareil (100) selon la revendication 1 comprenant en outre une mémoire numérique mémorisant un logiciel exécutable opérant avec le processeur (109) pour amener le processeur (109) à être fonctionnel pour coordonner la distribution du liquide (302) en direction de l'œil (102) sur la base d'une détection d'une lumière réfléchie par un œil (105).

3. Appareil (100) selon la revendication 2 comprenant en outre un capteur de proximité (108) en communication logique avec le processeur (109), ledit capteur de proximité (108) communiquant un signal logique sur la base des capteurs à proximité d'une surface.

4. Appareil (100) selon la revendication 3 comprenant en outre une buse de distribution (301) en communication fluidique avec le distributeur automatisé et fonctionnelle pour distribuer un liquide (302) par le biais d'une pulvérisation.

5. Appareil (100) selon la revendication 3 comprenant en outre une buse de distribution (301) en communication fluidique avec le distributeur automatisé et fonctionnelle pour distribuer un liquide (302) par le biais d'un brouillard.

6. Appareil (100) selon l'une quelconque des revendications 3 à 5 comprenant en outre un dispositif de signalisation sonore en communication électrique avec le capteur de proximité (108), ledit dispositif de signalisation sonore étant susceptible d'émettre un signal sonore sur la base d'un signal provenant du capteur (108).

7. Appareil (100) selon l'une quelconque des revendications 3 à 6, dans lequel le processeur (109) est fonctionnel pour recevoir un signal en provenance du détecteur (102) indiquant qu'un œil (105) est dans un état ouvert et pour générer un signal logique vers le distributeur sur la base de la réception du signal indiquant qu'un œil (105) est dans un état ouvert.

8. Appareil (100) selon la revendication 7 dans lequel le distributeur distribue un liquide (302) sur la base de la réception du signal indiquant qu'un œil (105) est dans un état ouvert.
